(19) Europäisches Patentamt
European Patent Office
Office européen des brevets

(11) Numéro de publication : **0 470 019 A1**

# (12) DEMANDE DE BREVET EUROPEEN

(21) Numéro de dépôt : **91420282.5**

(22) Date de dépôt : **30.07.91**

(51) Int. Cl.$^5$ : **A61K 31/80,** A61K 7/48

(30) Priorité : **31.07.90 FR 9010010**

(43) Date de publication de la demande :
**05.02.92 Bulletin 92/06**

(84) Etats contractants désignés :
**AT BE CH DE DK ES FR GB GR IT LI LU NL SE**

(71) Demandeur : **RHONE-POULENC CHIMIE**
**25, quai Paul Doumer**
**F-92408 Courbevoie Cédex (FR)**

(72) Inventeur : **Molteni, Carlo**
**Via Dei Bognetti, no. 9**
**I-20141 Milano (IT)**

(74) Mandataire : **Fabre, Madeleine-France et al**
**RHONE-POULENC CHIMIE Direction des Brevets Secteur Spécialités Chimiques 25, quai Paul-Doumer**
**F-92408 Courbevoie Cédex (FR)**

## (54) Crème adoucissante pour la peau et les gencives.

(57) La présente invention concerne une crème adoucissante pour la peau et les gencives, pour calmer en particulier les brulûres et les hématomes, caractérisée en ce qu'elle comporte :

A. - 100 parties en poids d'une huile silicone sensiblement linéaire de formule :

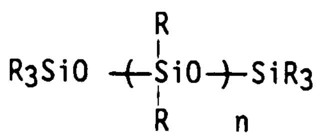

(1)

dans laquelle les radicaux R, identiques ou différents, sont choisis parmi un radical alkyle ayant de 1 à 10 atomes de carbone, un groupe vinyle et un groupe méthyle, n étant un nombre entier variant entre 5 et 300 inclus, de préférence entre 10 et 200 inclus ;

B. - 5 à 40, de préférence 10 à 20, parties en poids d'une huile silicone sensiblement linéaire hydroxylée de formule :

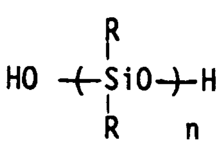

(2)

dans laquelle R a la même signification qu'à la formule (1) et n est un nombre entier variant entre 2 et 100 inclus, de préférence entre 5 et 20 inclus ; et

C. - 2 à 50, de préférence 10 à 30, parties en poids d'une charge siliceuse.

EP 0 470 019 A1

La présente invention concerne une crème adoucissante pour la peau humaine et les gencives, en particulier pour calmer les lésions telles que brûlures et hématomes.

Par le brevet français FR-A-1 503 572, il est connu de soulager (calmer) les brûlures de la peau en immergeant la partie brûlée dans un bain d'huile silicone non réactive.

Cette méthode, bien qu'efficace présente l'inconvénient d'être difficile à mettre en oeuvre et nécessite des quantités importantes d'huiles silicones.

Il est également connu d'appliquer sur la brûlure un élastomère silicone mousse ou un gel de silicone. EP-A-300 620 est une illustration de cet art antérieur. Ces pansements sont efficaces mais présentent l'inconvénient de notamment laisser échapper le liquide lymphatique dans le cas des brûlures.

Il est par ailleurs bien connu d'utiliser les huiles silicones non réactives dans certaines crèmes et onguents dermiques utilisés en cosmétologie.

La présente invention a pour but de proposer une crème adoucissante pour la peau et les gencives, facile à appliquer, et plus particulièrement utilisable pour recouvrir les lésions dues aux brûlures et aux hématomes. S'agissant de la peau, les brûlures en question peuvent être d'origine solaire.

La crème selon l'invention comporte :

A. - 100 parties en poids d'une huile silicone sensiblement linéaire de formule :

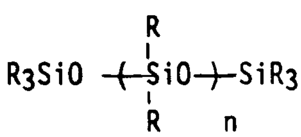
(1)

dans laquelle les radicaux R, identiques ou différents, sont choisis parmi un radical alkyle ayant de 1 à 10 atomes de carbone, un groupe vinyle et un groupe méthyle, n étant un nombre entier variant entre 5 et 300 inclus, de préférence entre 10 et 200 inclus ;

B. - 5 à 40, de préférence 10 à 20, parties en poids d'une huile silicone sensiblement linéaire hydroxylée de formule :

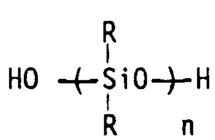
(2)

dans laquelle R a la même signification qu'à la formule (1) et n est un nombre entier variant entre 2 et 100 inclus, de préférence entre 5 et 20 inclus ; et

C. - 2 à 50, de préférence 10 à 30, parties en poids d'une charge siliceuse.

Par huiles A et B sensiblement linéaires on entend des huiles constituées principalement d'une succession de motifs diorganosiloxy ($R_2SiO_{2/2}$), toutefois la présence dans leur chaîne de motifs monoorganosiloxy ($RSiO_{3/2}$) ou silicate ($SiO_{4/2}$) n'est pas exclue à concurrence d'au plus 10 % molaire de motifs $RSiO_{3/2}$ et $SiO_{4/2}$ par molécule.

De préférence au moins 80 % en nombre de radicaux R sont méthyle.

Les charges siliceuses sont choisies parmi les charges siliceuses naturelles broyées telles que les terres de diatomées, et les silices de précipitation ou de combustion utilisées habituellement comme charge dans les élastomères silicones. Les silices de combustion sont les charges préférées.

L'huile B est le constituant actif de la crème. On a en effet trouvé que la présence des fonctions silanol sont à l'origine de l'action calmante et adoucissante de la crème sur les lésions de la peau et des gencives.

On a par ailleurs constaté que, dans le cas des brûlures, la crème selon l'invention permet d'étaler le liquide lymphatique s'écoulant de la brûlure et d'éviter son évaporation. La crème évite en outre la formation de cloques.

L'exemple ci-après, donné à titre indicatif, illustre l'invention et montre de quelle manière elle peut être mise en pratique

## EXEMPLE

On prépare dans un malaxeur une crème par mélange de :

A. - 79 parties d'une huile polydiméthylsiloxane bloquée à chacune de ses extrémités par un motif triméthylsilyle de viscosité 350 mPa.s à 25 °C, comportant environ en moyenne 100 motifs diméthylsiloxy,

B. - 12 parties d'une huile (a,ω-dihydroxy)polydiméthylsiloxane de viscosité 50 mPa.s à 25 °C, comportant en moyenne environ 6 motifs diméthylsiloxy,

C. - 9 parties d'une silice de combustion non traitée de surface spécifique BET de 130 $m^2/g$.

On obtient une crème onctueuse que l'on conditionne, dans des conditions optimales de pratique de fabrication, à l'abri de l'air dans des tubes, genre tube de dentifrice.

Appliquée sur une brûlure de la peau, la crème a une action calmante sur la douleur et évite la formation de cloques.

## EXEMPLE COMPARATIF :

On refait exactement le mode opératoire de l'exemple sauf que l'on n'ajoute pas l'huile B. La crème obtenue présente un effet calmant, sur la même brûlure, très inférieur.

## Revendications

1. Crème adoucissante pour la peau et les gencives, utilisable en particulier sur les brûlures et les hématomes, caractérisée en ce qu'elle comporte :

   A. - 100 parties en poids d'une huile silicone sensiblement linéaire de formule :

$$R_3SiO \left( \begin{array}{c} R \\ | \\ SiO \\ | \\ R \end{array} \right)_n SiR_3 \qquad (1)$$

   dans laquelle les radicaux R, identiques ou différents, sont choisis parmi un radical alkyle ayant de 1 à 10 atomes de carbone, un groupe vinyle et un groupe méthyle, n étant un nombre entier variant entre 5 et 300 inclus, de préférence entre 10 et 200 inclus ;

   B. - 5 à 40, de préférence 10 à 20, parties en poids d'une huile silicone sensiblement linéaire hydroxylée de formule :

$$HO \left( \begin{array}{c} R \\ | \\ SiO \\ | \\ R \end{array} \right)_n H \qquad (2)$$

   dans laquelle R a la même signification qu'à la formule (1) et n est un nombre entier variant entre 2 et 100 inclus, de préférence entre 5 et 20 inclus ; et

   C. - 2 à 50, de préférence 10 à 30, parties en poids d'une charge siliceuse.

Office européen des brevets

# RAPPORT DE RECHERCHE EUROPEENNE

Numero de la demande

EP 91 42 0282

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 268 950 (BAYER AG)<br>* Revendications *<br>--- | 1 | A 61 K 31/80<br>A 61 K 7/48 |
| A | EP-A-0 133 964 (REVLON INC.)<br>* Revendication 1 *<br>----- | 1 | |

| DOMAINES TECHNIQUES RECHERCHES (Int. Cl.5) |
|---|
| A 61 K |

Le présent rapport a été établi pour toutes les revendications

| Lieu de la recherche | Date d'achèvement de la recherche | Examinateur |
|---|---|---|
| LA HAYE | 04-11-1991 | LEHERTE C.F.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)